# EUROPEAN PATENT APPLICATION

(11) **EP 4 706 712 A2**
(43) Date of publication of application: **11.03.2026**
(21) Application number: 25196670.1
(22) Date of filing: 19.08.2025
(51) Int. Cl.: A61M 16/00, A61B 1/267, A61B 50/13, A61M 16/01

(54) **MEDICAL DEVICE INTEGRATION WITH ANESTHESIA MACHINE**

(30) Priority: 10.09.2024 US 202418829670
(71) Applicant: GE Precision Healthcare LLC, Waukesha, WI 53188 (US)
(72) Inventor: RINDY, Ryan, Waukesha, 53188-1696 (US); BAUM, Bradley, Waukesha, 53188-1696 (US); CHRISTMAN, Thomas, Waukesha, 53188-1696 (US)
(74) Representative: Kilburn & Strode LLP

(57) **Abstract**

An anesthesia machine that integrates the presence and charge status of another medical device, such as a video laryngoscope, into the operation. The anesthesia machine communicates with the medical device to determine the presence and charge status and present this information to the user as part of the checkout procedure. A medical device holder is associated with the anesthesia machine to receive and charge the medical device. The medical device holder can include both a medical device charging holder and a battery charging holder that receives and charges a replacement battery. The medical device holder can communicate with the anesthesia machine to provide the charge and presence status of the medical device. During the checkout procedure for the anesthesia machine, the user can be presented with the presence and battery status information or could be prompted to manually enter this information into the anesthesia machine.

## Description

### BACKGROUND

The present disclosure generally relates to an anesthesia machine that can be integrated with other medical devices, such as a video laryngoscope. More specifically, the present disclosure relates to the integration of the video laryngoscope with the anesthesia machine such that functional characteristics of the video laryngoscope can be detected from the anesthesia machine.

Anesthesia machines are currently available that can include a movable cart that includes a main body and a variety of pieces of equipment that are used to administer anesthesia to a patient and for monitoring the vital signs of the patient during a procedure. One example of an anesthesia machine is the Carestation^{™} Anesthesia Delivery System available from GE HealthCare. This anesthesia machine includes one or more display screens that allow a clinician to control the operation parameters of the delivery components while visually monitoring the vital signs of the patient during the procedure. Other anesthesia systems exist that can include wall mounted or pendant mounted equipment.

Presently, the use of a video laryngoscope (VL) is becoming standard practice for intubating patients prior to ventilation and the provision of anesthetic agents to the patient. The video laryngoscope has many benefits during the intubation procedure and creates a new workflow in the anesthesia process. Currently available video laryngoscopes, such as the McGrath^{™} MAC video laryngoscope available from Medtronic, include disposable blades connected to a flexible handle and a visual display that allows the clinician to view the procedure. The video laryngoscope is a wireless device that includes a rechargeable battery and one or more replacement batteries. The video laryngoscope includes a battery indicator that can be viewed from the device. In some clinical environments, the video laryngoscope is stored in a wall mounted holder/charger that can be located in an operating room or within a operating theatre. In other situations, the video laryngoscope is mounted to a movable cart and can be moved from location to location. Additionally, video laryngoscopes are available that are cart based and include a wired connection between the cart and the laryngoscope. Cart-based units are becoming less and less utilized as wireless devices are becoming more and more popular.

Before beginning a procedure using the anesthesia machine, the clinician often must search out the video laryngoscope from another room or location. This can cause a delay in the anesthesia procedure or if the video laryngoscope cannot be located, the procedure is carried out with an available laryngoscope that does not include the video display.

In other cases, even if the video laryngoscope is located, the clinician is not aware of the charge status of the internal battery until the video laryngoscope is obtained. In such a situation, the clinician will need to either recharge the battery installed in the equipment or search out replacement batteries and hope the replacement battery has a sufficient charge.

In addition to the video laryngoscope, other medical devices can also be used during procedures that are carried out with the anesthesia machine. For example, portable ultrasound devices and portable patient monitoring can be used in concert with the anesthesia machine. These medical devices also include rechargeable batteries that need to be charged, and the medical device located prior to the anesthesia procedure, similar to the problems that can occur with the use of the video laryngoscope.

The inventors recognized the need for a better method and system for locating and integrating a medical device, such as a video laryngoscope, with an anesthesia machine. In accordance with the present disclosure, the medical device communicates with the anesthesia machine so that information about the battery status and the presence of the medical device can be part of the checkout procedure and process for the anesthesia machine.

### SUMMARY

This Summary is provided to introduce a selection of concepts that are further described below in the Detailed Description. This Summary is not intended to identify key or essential features of the claimed subject matter, nor is it intended to be used as an aid in limiting the scope of the claimed subject matter.

In one embodiment, an anesthesia machine is provided that is able to deliver anesthesia to a patient and is usable in concert with a medical device, such as but not limited to a video laryngoscope, an ultrasound probe and a portable patient monitor. The anesthesia machine is able to coordinate the operation of the medical device with the operation of the anesthesia machine and include the medical device in the checkout procedure for the anesthesia machine.

The anesthesia machine includes a main body and a display screen for presenting information to the clinician or user. In one contemplated embodiment, the anesthesia machine includes a medical device holder that is associated with the main body of the machine. The medical device holder is configured to receive the medical device and charge the medical device when the medical device is received in the medical device holder. In one embodiment, the medical device communicates with the anesthesia machine such that the anesthesia machine can display status information about the medical device. It is contemplated that the status information can include the presence of the medical device, the charge status of the medical device and function information about the device. The function information received from the medical device could include the results of a self-test procedure that confirms all of the systems of the medical device are operating properly. The additional functional information from the medical device could be received and displayed.

In one exemplary embodiment, the medical device holder can be an insert received within a drawer that is movable into and out of the main body of the anesthesia machine. The insert within the drawer is connected to the power management board of the anesthesia machine such that the insert received power from the anesthesia machine. The insert can include a device charging holder that is designed to receive the specific medical device being used with the anesthesia machine. As an example, if the medical device is a video laryngoscope, the medical device holder would be physically configured to receive and hold the video laryngoscope. The medical device holder can further be configured to charge the medical device when the medical device is received in the medical device holder. This charging can take place utilizing inductive charging or by creating a wired connection between the insert and the medical device. When the medical device is received in the medical device holder, the medical device holder can communicate both the presence of the medical device and the state of charge to the control unit of the anesthesia machine.

In addition to the medical device holder, the insert can also include one or more battery receptacles that are each designed to receive a replacement battery for the medical device. When the replacement batteries are received in the battery receptacles, the replacement batteries are charged, and the presence of the replacement battery is communicated to the control unit of the anesthesia machine.

In accordance with another aspect of the present disclosure, a medical device holder is provided for use with an anesthesia machine that includes a main body and a display screen. The medical device holder includes a power connection to receive electrical power from the anesthesia machine. The medical device holder further includes a device charging holder that is specifically configured to receive the medical device and charge the medical device while the medical device is received by the charging holder. In a contemplated exemplary embodiment, the medical device holder can further include one or more battery charging receptacles that are configured to receive and charge replacement batteries for the medical device. The charging device holder and the battery charging receptacles can be configured to charge the batteries of the medical device and the replacement batteries utilizing either an inductive coupling or a plug connection.

The medical device holder is configured to provide status information about the medical device to the anesthesia machine. This status information can include the state of charge of the medical device and the presence of the medical device in the medical device holder. The status information received by the anesthesia machine can be displayed and form part of the checkout procedure for the anesthesia machine. In addition, the status information could also be displayed on a remotely located dashboard, which would allow a biomed department/anesthesia director to see and monitor the status of all anesthesia machines and video laryngoscopes in the hospital or healthcare facility.

In one contemplated exemplary embodiment, the medical device holder is formed as an insert received in a drawer of the main body of the anesthesia machine. The insert received in the drawer includes the charging device holder and the battery receptacles that are each coupled to the power supply board of the anesthesia machine.

The present disclosure also provides a method of operating an anesthesia machine in concert with a medical device during the performance of an anesthesia procedure. The method includes the step of determining the charge status of the medical device. The charge status can be determined by a wired or wireless communication from the medical device or by a communication from a medical device holder associated with the anesthesia machine. In addition to the charge status, the method determines the presence of the medical device as part of the checkout procedure for the anesthesia machine. The presence of the medical device can be determined by a physical switching element included in the medical device holder or by some other interaction between the medical device holder, such as charging of the medical device.

Once the charge status and the presence status are determined, the status is displayed on the anesthesia machine for viewing and interaction by the clinician. The status is presented as part of the checkout procedure for the anesthesia machine and is another step taken by the clinician prior to starting a procedure.

In one exemplary embodiment, the charge status and presence status are determined automatically either by a wired or wireless communication between the anesthesia machine and the medical device. Alternatively, the clinician can be presented with manual prompts for the clinician to respond to and enter the status information. The manual prompts require action by the clinician and ensure the medical device is present and ready to use as part of the anesthesia procedure.

Various other features, objects, and advantages of the invention will be made apparent from the following description taken together with the drawings.

### BRIEF DESCRIPTION OF THE DRAWINGS

The present disclosure is described with reference to the following Figures.
Fig. 1 is a front perspective view of an anesthesia machine having a movable cart including a main body, a series of devices and a display screen;
Fig. 2 is a front perspective view of the anesthesia machine with one of the drawers open showing the insert that receives and charges a medical device and one or more replacement batteries;
Fig. 3 is a magnified view of the open drawer and insert of Fig. 2
Fig. 4 is a view of a video laryngoscope that can be used with the anesthesia machine of Fig. 1;
Fig. 5 is an exemplary checkout screen of the anesthesia machine;
Fig. 6 is an exemplary dashboard screen for the anesthesia machine including status information for the medical device;
Fig. 7 is a magnified view of an alternate embodiment for the placement of the medical device holder;
Fig. 8 is a view of an ultrasound probe that can be used with the anesthesia machine;
Fig. 9 is a view of a portable patient monitoring device that can be used with the anesthesia machine;
Fig. 10 is a schematic illustration of one type of communication architecture between the anesthesia machine and the medical device;
Fig. 11 is a schematic illustration of a first alternate type of communication architecture between the anesthesia machine and the medical device;
Fig. 12 is a schematic illustration of a second alternate type of communication architecture between the anesthesia machine and the medical device;
Fig. 13 is a schematic illustration of a third alternate type of communication architecture between the anesthesia machine and the medical device; and
Fig. 14 is a flow chart illustrating one method of operating the anesthesia control system with a medical device.

### DETAILED DESCRIPTION

The present inventors have recognized several problems with currently available anesthesia machines when utilized with other medical devices, such as a video laryngoscope, during a procedure that integrates the use of both the anesthesia machine and the medical device. Presently, when the clinician is completing a checkout procedure for the anesthesia machine, the clinician does not receive any information about the location and status of the ancillary medical device. Thus, the clinician must remember to search for the medical device and check the charge status of the medical device separate from the checkout procedure for the anesthesia machine. The present disclosure was developed by the inventors to integrate the checkout procedure for the anesthesia machine with the presence status and charging status of the medical device to streamline the procedure and enhance the functionality of the anesthesia machine.

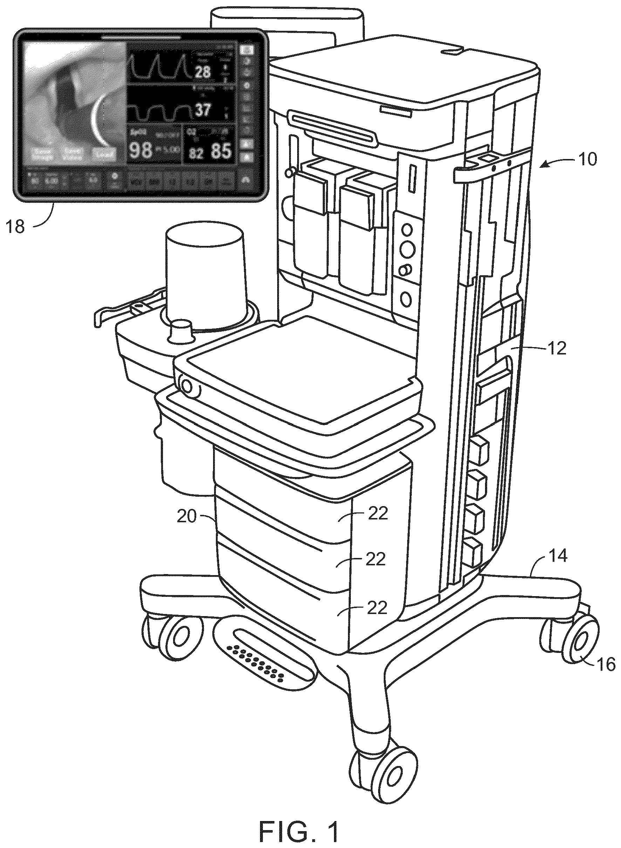

Fig. 1 illustrates an anesthesia machine 10 constructed in accordance with one embodiment of the present disclosure. Anesthesia machine 10 takes the form of a moveable cart that includes a main body 12 that is supported on a base 14 that includes a plurality of wheels 16 that allow the entire anesthesia machine 10 to be moved around in a care environment. The main body 12 supports a series of operating components that are used to deliver an anesthetic agent to a patient and to ventilate the patient as is desired. An illustrative example of the anesthesia machine 10 is the Care Station ^{™} 600 that is available from GE Healthcare. It should be understood that various other different types of anesthesia machines 10 could be utilized while operating within the scope of the present disclosure. The anesthesia machines 10 could be either mobile units, ceiling pendant mounted, or wall mounted depending on the hospital or healthcare facility.

The anesthesia machine 10 includes at least one display screen 18 that is used to present information to a clinician. In addition, the display screen 18 can include a touch screen that allows the clinician to enter information, modify operational parameters and otherwise control the overall operation of the anesthesia machine 10. The details of the operation of the anesthesia machine 10 are well-known to those of ordinary skill in the art.

In the embodiment shown in Fig. 1, the anesthesia machine 10 includes a storage cabinet 20 that is mounted to the front of the main body 12. The storage cabinet 20 provides an area to store different types of components necessary to utilize the anesthesia machine during a procedure. In the embodiment shown in Fig. 1, the storage cabinet 20 includes three separate drawers 22 that can each be independently opened to provide access to the open interior of the drawer 22.

As described previously, the use of the anesthesia machine 10 with other types of medical devices to enhance the operation of the anesthesia machine 10 during a patient procedure is becoming more common. As an example, the anesthesia machine 10 can be utilized with a variety of different types of medical devices, such as but not limited to a video laryngoscope (VL), a portable ultrasound probe, a portable patient monitoring device or any other type of medical devices that is both portable and self-contained and can be used during a procedure that otherwise utilizes the anesthesia machine 10. During the preparation for the performance of a procedure that utilizes the anesthesia machine 10 and the auxiliary medical device, a clinician must first locate the medical device prior to beginning the procedure. In many cases, the clinician may not remember to locate the medical device before beginning the procedure and would need to search out the medical device before beginning the medical procedure utilizing the anesthesia machine. Further, even when the medical device is located, the clinician must remember to check the charge status of the medical device and retrieve additional replacement batteries if the procedure is going to occur over an extended period of time.

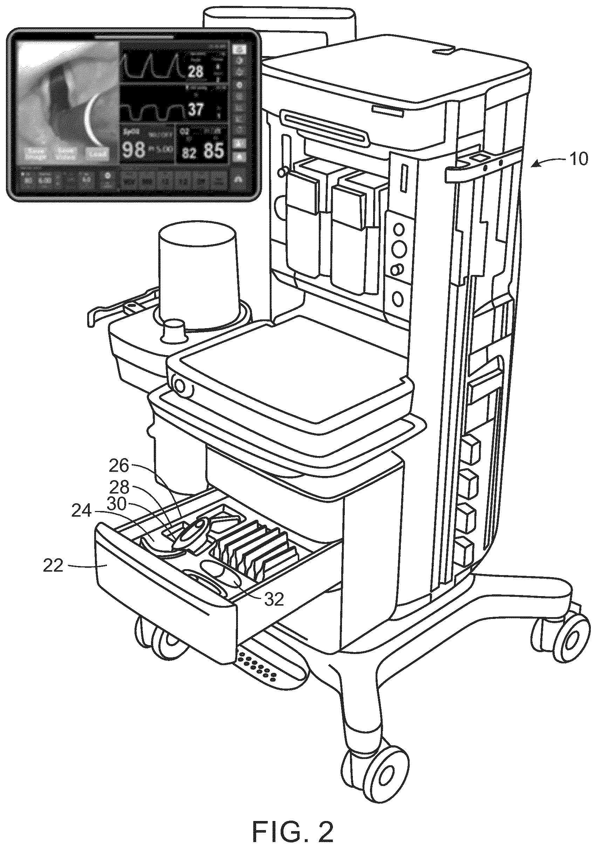

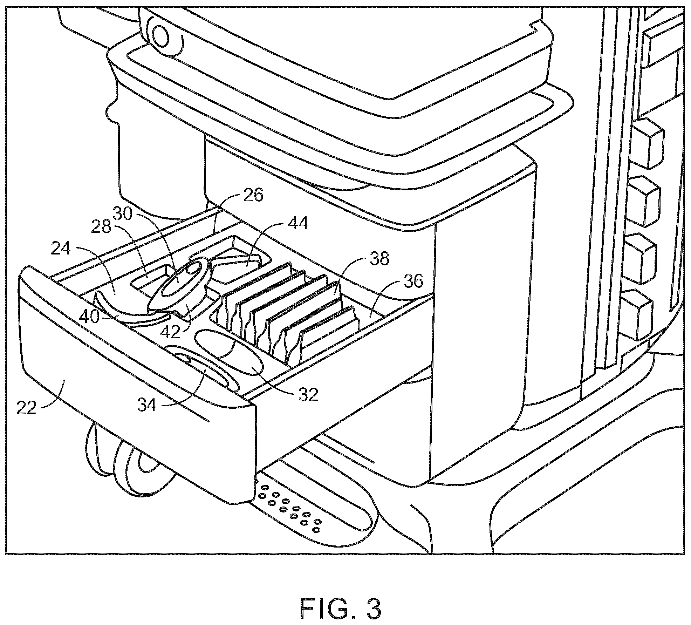

Referring now to Figs. 2 and 3, in accordance with the present disclosure, one or more of the drawers 22 is configured to include a medical device holder 24 that is used to not only store the medical device but also provide charging for the medical device when the medical device is stored during non-use within the medical device holder 24. In the embodiment shown, the medical device holder 24 takes the shape of an insert 26 that is sized to be received within the open interior of the drawer 22. The insert 26 includes a device charging holder 28 specifically sized to receive the medical device, such as the video laryngoscope 30. In the embodiment illustrated, the medical device will be shown as a video laryngoscope 30, although other medical devices are contemplated.

When the video laryngoscope 30 is received within the device charging holder 28, the insert 26 both recognizes the presence of the video laryngoscope 30 and charges the internal battery of the video laryngoscope 30 through inductive charging. Although the embodiment shown in Fig. 2 includes an insert 26 designed to receive a certain type of medical device, such as the video laryngoscope 30, it should be understood that the insert 26 could be designed to receive a variety of different types of medical devices, such as an ultrasound probe or patient monitoring device. Since the video laryngoscope 30 is becoming more and more commonly used with the anesthesia machine 10 during the performance of an anesthesia procedure, the insert 26 shown in Figs. 2 and 3 is specifically designed to receive the video laryngoscope 30. In addition to the device charging holder 28, the insert 26 includes a pair of battery charging receptacles 32 that are each specifically designed to receive a replacement battery 34. Each of the battery charging receptacles 32 is designed to allow inductive coupling and charging of the replacement battery 34 when the replacement battery 34 is inserted into the battery charging receptacle 32.

In addition to the device charging holder 28 and the battery charging receptacles 32, the insert 26 also includes a storage area 36 that is designed to receive and hold a variety of different supplies 38 that are required for use of the medical device. In the embodiment shown, the supplies 38 could be replacement disposable blades that are designed to incorporate into the flexible handle of the video laryngoscope 30. The replaceable blades 40 and other supplies are stored within the storage area 36 such that a clinician should hopefully have all of the supplies needed to use the video laryngoscope 30 during an anesthesia procedure.

It is contemplated that the entire drawer 22 or just drawer insert 26 could be in communication with the power management board (PMB) of the anesthesia machine such that the entire drawer 22 or the drawer insert 26 would receive power from the anesthesia machine and would be able to communicate information back to the PMB through a power/communication cable (not shown). The power communication between the drawer insert 26 and the PMB of the anesthesia machine will allow charging of the video laryngoscope 30 and the replacement batteries 34. The power/communication cable will also provide a wired communication path for information to be relayed from the device charging holder 28 and the battery charging receptacles 32 back to the control unit of the anesthesia machine. Specifically, it is contemplated that the communication between the drawer insert 26 and the anesthesia machine would provide information as to the current state of charge for the battery included in the video laryngoscope 30, confirmation of the presence of the video laryngoscope 30 within the device charging holder 28, the presence of replacement batteries 34 and the charge on each of the replacement batteries 34 that are received within the battery charging receptacles 32. The presence of one or more replacement batteries 34 can also be relayed to the anesthesia device.

It is contemplated that the device charging holder 28 could detect the presence of the video laryngoscope through either a physical switch contact or by the flow of charging power from the insert 26 to the video laryngoscope 30. Likewise, the presence of the replacement batteries 34 can be detected either through a physical switch contact or by the flow of electricity to one or both of the replacement batteries 34 when the replacement batteries 34 are received within the battery charging receptacle 32. In either case, the control unit of the anesthesia machine 10 would be able to detect the presence of the medical device and one or more of the replacement batteries as well as determine the current state of charge on the medical device and the replacement battery or batteries 34.

Since the insert 26 in an exemplary embodiment receives electric power to provide charging for both the video laryngoscope 30 and the replacement battery 34, the status information concerning the presence of the video laryngoscope 30 and the charging status are preferably relayed to the operating control unit of the anesthesia machine through a wired connection. However, it is contemplated that the battery status information from the video laryngoscope 30 and the replacement battery 34 could be relayed to the controls of the anesthesia machine using various different types of wired or wireless communication methods, such as but not limited to NFC, Bluetooth, IR, a physical USB connection or any other type of wireless or wired communication between the control unit for the anesthesia machine and the video laryngoscope 30 and the battery charging receptacle 32.

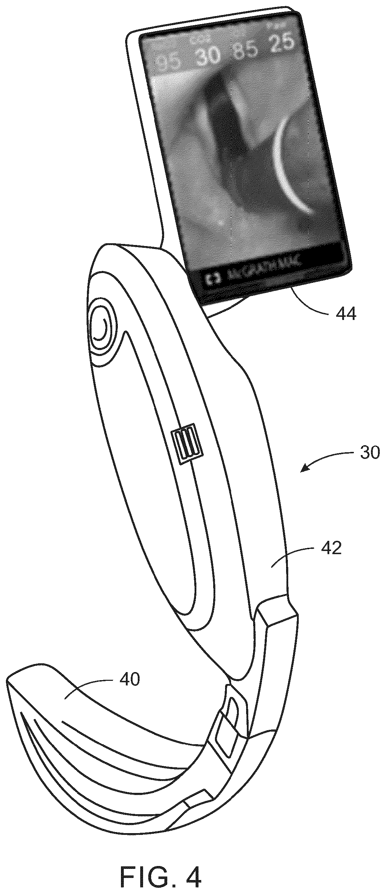

Referring now to Fig. 4, one exemplary embodiment of video laryngoscope 30 is illustrated. The video laryngoscope 30 includes one of the replaceable blades 40 connected to a flexible handle 42 that is utilized by the clinician to intubate the patient. The video laryngoscope 30 further includes a display screen 44 that presents an image to the clinician showing the intubation status as the clinician intubates the patient. One exemplary embodiment of the video laryngoscope is the McGrath^{™} MAC Video Laryngoscope that is available from MedTronic. However, it should be understood that various other different types of video laryngoscopes 30 could be utilized while operating within the scope of the present disclosure. The video laryngoscope 30 includes an internal controller and components that control the operation of the medical device and provide wireless communication to and from the device. The video laryngoscope 30 includes inductive charging capabilities such that the battery currently included in the medical device can be inductively charged without any physical connection.

As can be seen in the view of Fig. 3, the device charging holder 28 is specifically designed to receive the replaceable blade 40, flexible handle 42 and the display screen 44 of the video laryngoscope 30. If a different type of video laryngoscope 30 is utilized, the specific configuration of the device charging holder 28 can be modified to provide not only the safe and secure storage of the video laryngoscope 30 but also the proper charging of the device when it is received within the drawer 22. In a medical device that does not support inductive charging, other types of charging interfaces could be incorporated into the device charging holder 28. In some cases, a physical plug connection may be required to support the charging in the device charging holder 28.

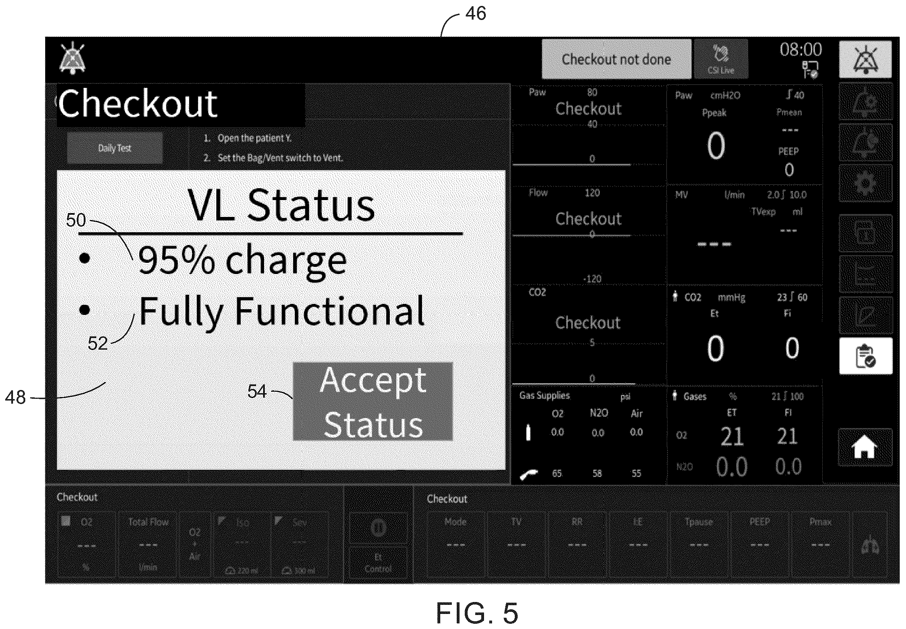

As indicated above, when the video laryngoscope 30 is received within the insert 26 located within the drawer 22, the insert 26 is able to determine the presence of the video laryngoscope 30 and relay the charge status to the control unit of the anesthesia machine. During the initial checkout process of the anesthesia machine 10, the clinician is presented with a checkout screen 46 similar to the screen shown in Fig. 5. The checkout screen 46 provides automated prompts that require the clinician to interact with the anesthesia machine to make sure all of the operating components of the anesthesia machine 10 are operating properly and that the anesthesia machine is primed and ready to be used during an anesthesia procedure. In accordance with the present disclosure, the checkout screen 46 is modified to include an additional visual prompt to the clinician regarding the presence and charging status of the medical device. As indicated previously, in the embodiment illustrated, the medical device is a video laryngoscope.

On the checkout screen 46 shown in Fig. 5, a VL status window 48 is presented to the clinician. The VL status window 48 includes the charging status 50 and the operational status 52 for the video laryngoscope. The charging status 50 also provides an indication of the presence of the video laryngoscope within the insert or in a close proximity to the anesthesia machine. The VL status window 48 includes an acceptance button 54 that prompts the clinician to depress the acceptance button 54, thereby acknowledging both the current state of charge 50 and the functionality status 52 for the video laryngoscope. The acceptance button 54 thus creates another step in the checkout process that is specifically related to the presence and status of the medical device, such as the video laryngoscope 30.

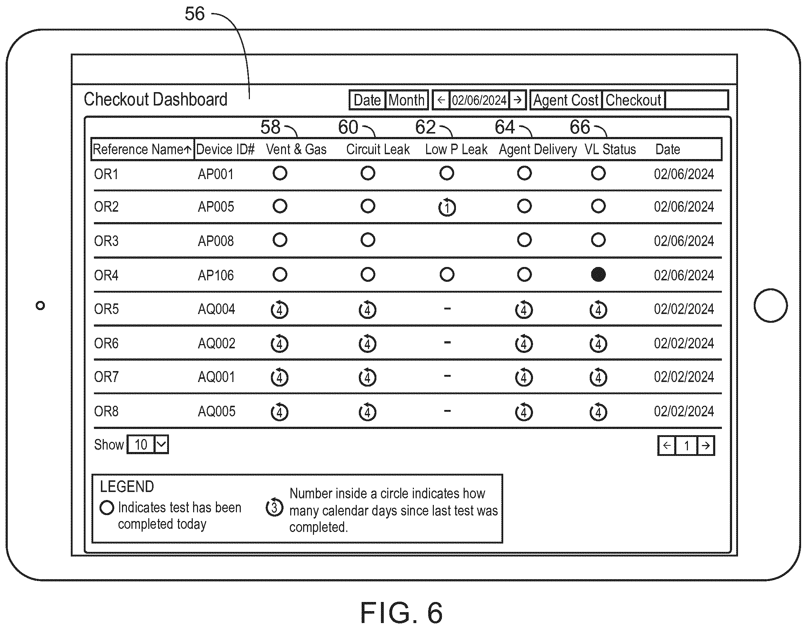

Fig. 6 illustrates a checkout dashboard 56 for the anesthesia machine. The checkout dashboard 56 is currently included with many available anesthesia machines, such as those available from GE Healthcare. The checkout dashboard 56 could be associated with the anesthesia machine or could be located elsewhere in the hospital/healthcare facility, such as in the biomed department or anesthesia directors' office. The checkout dashboard 56 provides the current status of various different components of the anesthesia machine and the various tests that have been performed and whether the system has passed the checks and when the next check needs to be performed. The checkout dashboard 56 can be used to view the status of all of the anesthesia machines and video laryngoscopes in the hospital. The first column is for a vent and gas check, as illustrated by reference numeral 58. The second column 60 is for the testing of any circuit leaks, the third column 62 is to detect a low-pressure leak, the fourth column 64 is for testing the delivery of the anesthesia agent. Column 66 has been added to the checkout dashboard 56 and includes the VL status. The VL status provides the current status of the medical device being utilized with the anesthesia machine. As indicated previously, the VL status column 66 provides a status for the video laryngoscope that is used with the anesthesia machine. The VL status column 66 in the embodiment shown displays the status of the video laryngoscope that is going to be utilized with the anesthesia machine, which was determined as part of the checkout done by the clinician at the anesthesia machine. The inclusion of the VL status column 66 provides a convenient location for the clinician to confirm the operational status of the medical device as part of the checkout procedures for the anesthesia machine.

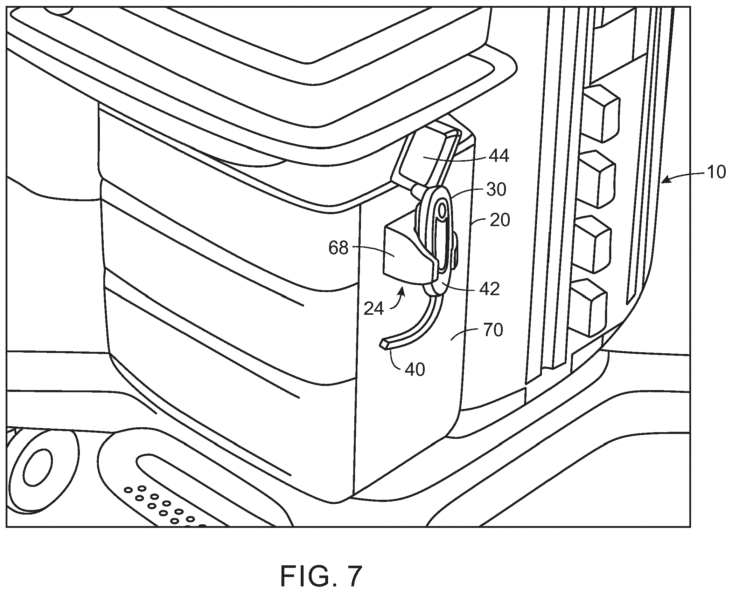

Referring now to Fig. 7, one possible alternate configuration for the medical device holder 24 is illustrated. In this embodiment, the medical device holder 24 takes the form of a charging cradle 68 that is mounted to side wall 70 of the storage cabinet 20. The charging cradle 68 can be connected to the internal power supply of the anesthesia machine through the power management board such that charging power could be provided to the charging cradle 68 to charge the medical device when it is received within the charging cradle 68. In the embodiment shown, the same video laryngoscope 30 is shown being received and retained with the charging cradle 68. The charging cradle 68 could also include a communication cable that would relay status information to the control unit of the anesthesia machine, including the presence of the video laryngoscope 30 and the current charge status on the internal battery contained within the flexible handle 42. Alternatively, the video laryngoscope 30 could communicate using a wireless communication protocol to relay information to the control unit of the anesthesia machine to provide both the presence information and the current charge status on the battery contained within the flexible handle 42.

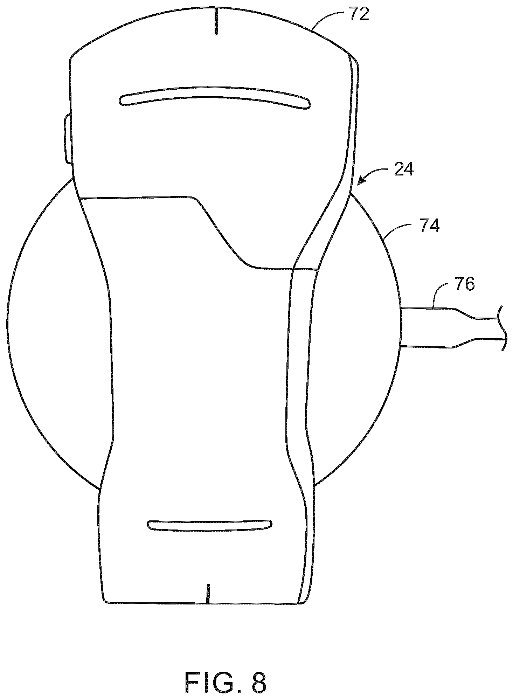

Fig. 8 illustrates yet another type of medical device that could be utilized within the scope of the present disclosure. In the embodiment shown, the medical device is an ultrasound probe 72 is shown mounted to a charging pad 74. The charging pad 74 could be mounted anywhere on the main body of the anesthesia machine. The charging pad 74 includes a power cord 76 that provides charging power for charging the ultrasound probe 72 when the ultrasound probe 72 is not in use. The power cord 76 could also include a communication wire that relays both the charge status and the presence status of the ultrasound probe 72 on the charging pad 74. In such an embodiment, the charging pad 74 would function as the medical device holder 24 that can be located anywhere on the main body of the anesthesia machine. Once again, it is contemplated that the ultrasound probe 72 could communicate with the anesthesia machine utilizing wireless communication protocols.

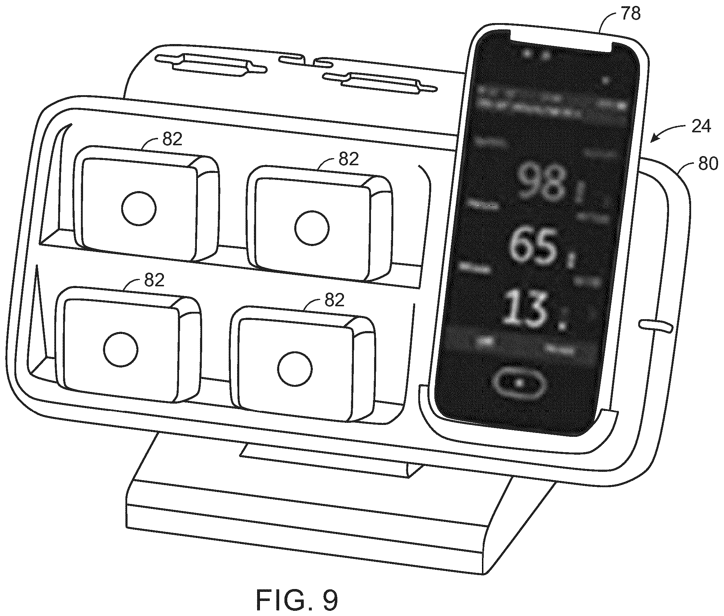

Fig. 9 illustrates yet another type of medical device that could be utilized with the anesthesia machine of the present disclosure. In the embodiment shown in Fig. 9, the medical device is a portable patient monitoring device 78 that is shown supported on a charging base 80. The charging base 80 can be connected to the power supply of the anesthesia machine through the power management board such that when the charging base 80 is installed on the anesthesia machine, charging power can be used for charging not only the portable patient monitoring device 78 but also a series of rechargeable sensor battery packs 82. The rechargeable sensor battery packs 82 can be utilized with a variety of different types of portable sensors (not shown) that can detect various different vital signs from a patient. The portable sensors relay the sensed vital sign information to the patient monitoring device 78 for review and analysis by a clinician. It is contemplated that the information received by the portable patient monitoring device could be relayed to the anesthesia machine and displayed on the display of the anesthesia machine. Such an embodiment is yet another source of information presented for review and analysis by the clinician during an anesthesia procedure.

Although various different types of medical devices, such as a video laryngograph, ultrasound probe and portable patient monitoring device are shown and described in accordance with the present disclosure, it should be understood that other types of medical devices could be utilized in concert with the operation of the anesthesia machine 10 while operating within the scope of the present disclosure.

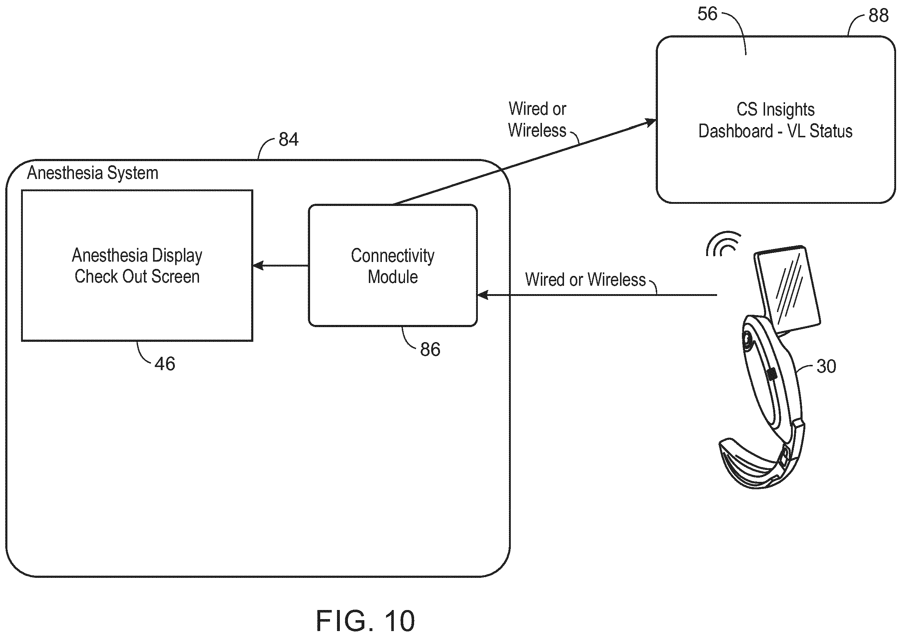

Referring now to Fig. 10, thereshown is a first exemplary embodiment of a possible communication architecture that can be utilized between the anesthesia control system 84 that is included in the anesthesia machine and is utilized to operate the anesthesia device and communicate with one or more medical devices. The anesthesia control system 84 is schematically shown as including the anesthesia checkout screen 46 that was previously shown in Fig. 5 and described. The anesthesia checkout screen 46 receives information from a connectivity module 86 that provides an interface between the display including the checkout screen 46 and both the medical device and the checkout dashboard 56. The checkout dashboard 56 was previously described with reference to Fig. 6.

In the embodiment shown in Fig. 10, the connectivity module 86 is able to communicate with the medical device, which is shown as the video laryngoscope 30, utilizing either a wired or wireless communication technique. The wired or wireless communication technique would be used in an embodiment such as shown in Fig. 7 in which the charging cradle 68 is mounted to a location on the storage cabinet 20. In an embodiment in which a communication cable extends between the charging cradle 68 and the anesthesia machine, a wired communication would be sent from the medical device 24 to the connectivity module 86. As described previously, this communication can include at least the presence status of the medical device, and the charge status of the battery installed in the medical device. In an embodiment in which a communication cable is not present, the medical device would be able to communicate to the control system 84 of the anesthesia machine utilizing a wireless protocol, such as NFC, Bluetooth, IR or any other type of available wireless communication technique.

As shown in Fig. 10, the connectivity module 86 is also able to communicate with an external display 88 that can include the checkout dashboard 56 previously described with reference to Fig. 6. Again, the communication from the connectivity module 86 to the external display 88 can include either a wired communication cable or a wireless communication protocol. In this manner, the connectivity module 86 will be able to receive information from the medical device and relay this information to the external display 88 for display in the VL status column 66 shown in the checkout dashboard 56 of Fig. 6. The checkout dashboard 56 can display the checkout status for multiple anesthesia machines and multiple medical devices and can be located in remote locations, such as in a biomed department or the office of the anesthesia direction of a hospital or healthcare facility.

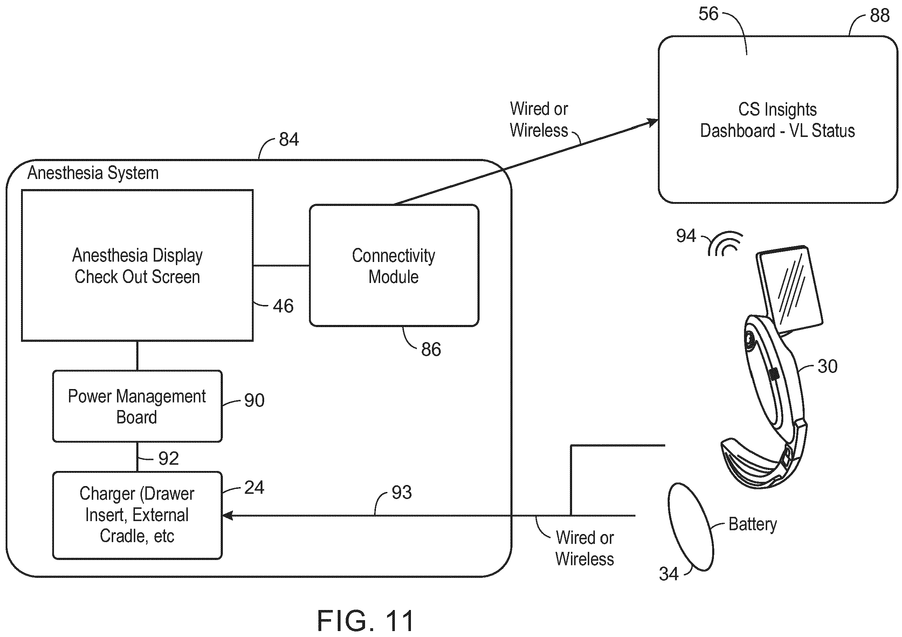

Fig. 11 illustrates a second contemplated communication protocol for communicating between the anesthesia control system 84, the medical device, one or more replacement batteries 34 and the external display 88 including the checkout dashboard 56. In the second embodiment of the communication technique shown in Fig. 11, the anesthesia display checkout screen 46 communicates with a power management board 90 that provides powered supply connections to a medical device holder 24. As described previously, the medical device holder 24 can be a drawer insert, an external cradle or any other type of charging device and/or communication device that is connected to the power management board 90 to receive power to charge a medical device that is received within the medical device holder 24. It is contemplated that a charging and communication wire 92 will extend between the power management board 90 and the medical device holder 24. In the embodiment shown, a wired connection 93 exists between the medical device holder 24 to the medical device (video laryngoscope 30) and the replacement battery 34 to provide powered charging abilities for both the video laryngoscope 30 and the replacement batteries 34. In addition to the electrical power to charge each of these two components, the wired connection 93 would also obtain charge status and presence status for both the video laryngoscope 30 and the replacement battery or batteries 34. As was the case in the other embodiments, the medical device could also communicate with the control system 84 of the anesthesia machine utilizing a wireless communication protocol.

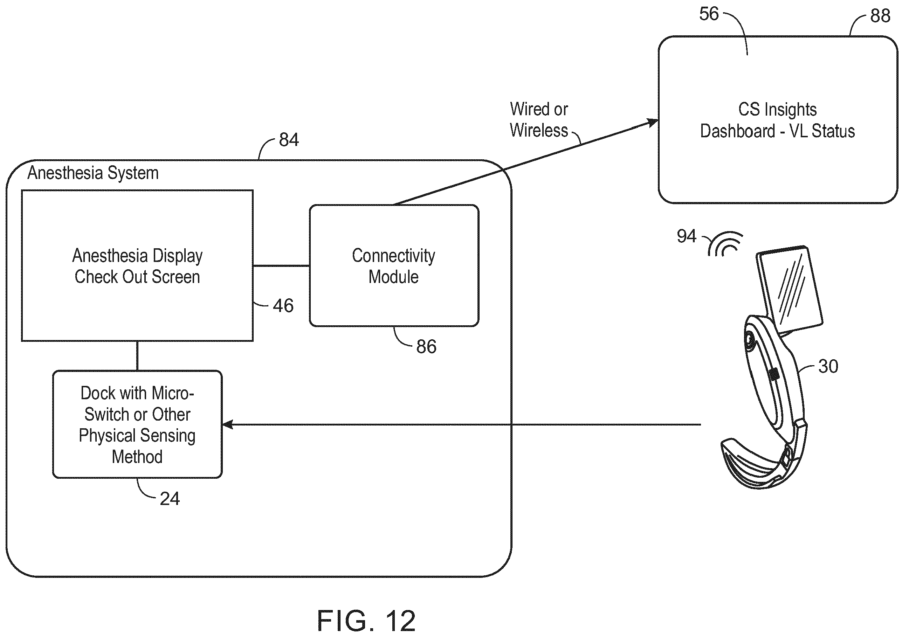

Fig. 12 illustrates yet another alternate type of communication protocol between the anesthesia control system 84 and the medical device, shown schematically by the video laryngoscope 30. In this embodiment, the medical device holder 24 can be a docking cradle or holder (see Figs. 7-9) that includes some type of microswitch or other physical-sensing mechanism that is able to detect the presence of the medical device, such as the video laryngoscope 30. In such an embodiment, the medical device holder 24 would include some type of physical switching device that can detect the presence of the medical device and relay this information to the anesthesia display checkout screen 46. In the embodiment shown, the video laryngoscope 30 can communicate with the anesthesia control system 84 utilizing a wireless communication technique 94. Such communication may be through the connectivity module 86. This wireless communication 94 would relay information about the operating status of the medical device, such as the current battery status level. Since the medical device holder 24 includes a physical switch to detect the presence of the medical device, the system of Fig. 12 utilizes both a physical switch connection and wireless communication techniques to detect the presence of the medical device and determine the charge status on the medical device. As in the previously described embodiments, the connectivity module 86 is also able to communicate with an external display 88 that can include the checkout dashboard 56 previously described with reference to Fig. 6. Again, the communication from the connectivity module 86 to the external display 88 can include either a wired communication cable or a wireless communication protocol. In this manner, the connectivity module 86 will be able to receive information from the medical device and the medical device holder 24 and relay this information to the external display 88 for display in the VL status column 66 shown in the checkout dashboard 56 of Fig. 6

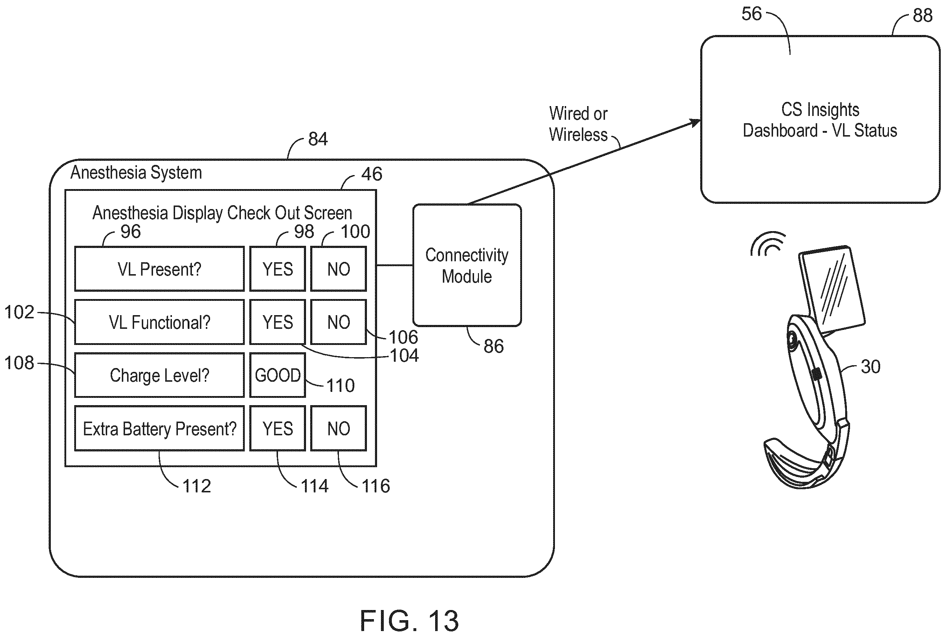

Fig. 13 illustrates yet another alternate contemplated communication protocol between the anesthesia control system 84 and the medical device shown by the video laryngoscope 30. In the embodiment shown in Fig. 13, the checkout screen 46 has been modified to include four separate prompts that require the clinician to manually enter information related to the medical device. This modification to the checkout screen 46 is designed for situations in which there is no communication between the medical device and the anesthesia machine and the system relies upon manual data entry from the clinician during the initial checkout procedure for the anesthesia machine.

As shown in Fig. 13, the first prompt 96 presented requires the clinician to enter a decision as to whether the medical device, such as the video laryngoscope, is present in the area around the anesthesia machine. The clinician can select either the yes button 98 or the no button 100 to provide an indication of whether the video laryngoscope is within the immediate area. This prompt thus requires the clinician to locate the medical device and confirm the presence before beginning the anesthesia procedure. The clinician is then presented with prompt 102 that inquires as to whether the video laryngoscope is functional. This prompt required the clinician to review the operation of the medical device and confirm that it is operating properly. Again, this requires the clinician to take steps before beginning a procedure to ensure that the medical device being used is operating properly. The clinician can select between the yes prompt 104 and the no prompt 106. If the video laryngoscope is functional, the anesthesia control system 84 will allow the system to proceed to prompt 108. If the video laryngoscope is either not functional or not present, the clinician could proceed with the procedure using a traditional laryngoscope. In prompt 108, the clinician is required to determine the charge level of the video laryngoscope 30 and indicate whether the charge status is acceptable, as shown by button 110. It is also contemplated that the charging prompt could be modified to allow the clinician to enter the charging percentage if this information is available to the clinician from the medical device.

The final prompt is prompt 112 which requires the clinician to indicate whether an extra battery is present. The clinician can select between an affirmative selection 114 or a negative selection 116. This prompt reminds the clinician to look for an extra replacement battery as part of the initial checkout procedure for the anesthesia machine.

In the communication technique shown in Fig. 13, the video laryngoscope 30 does not need to communicate directly with the connectivity module 86. The communication technique of Fig. 13 allows the anesthesia control system to work with older medical devices, such as the video laryngoscopes 30, that may not have communication capability or may include a charging location that is located remotely from where the anesthesia machine is being utilized. In addition, this technique can be used with anesthesia machines that do not have the ability to add a medical device holder that communicates with and charges a medical device. The technique shown in Fig. 13 requires the clinician to manually enter all of the information utilizing visual prompts presented to the clinician during the checkout procedure. The use of a manual checkout procedure ensures that the clinician has searched for and obtained the video laryngoscope 30 before the procedure and has determined the operating status of such video laryngoscope 30.

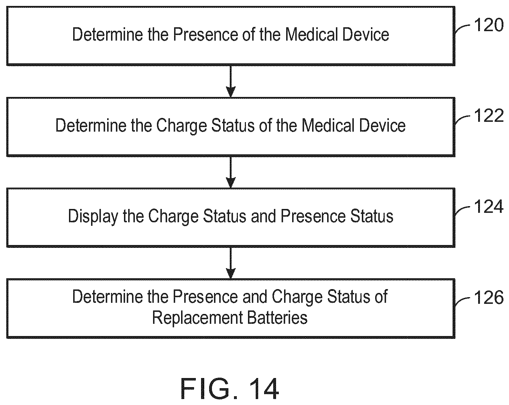

Referring now to Fig. 14, one exemplary method of operating the anesthesia machine shown and described in the previous figures will now be discussed. As indicated previously, the first step 120 in the method is for the anesthesia control system to initially determine whether the medical device is present. As discussed in the embodiments shown in Figs. 10-13, the anesthesia control system can determine the presence of the medical device utilizing either a physical switch, a wireless communication protocol with the medical device, or receiving a positive manual entry from the clinician in response to the prompt 96 shown in Fig. 13.

In step 122, the anesthesia control system determines the charge status of the medical device. Once again, the charge status of the medical device can be determined utilizing either a wired connection to the medical device, a wireless connection to the medical device or based upon the information entered by the clinician in response to prompt 108 in the manual embodiment of Fig. 13. Once the anesthesia control system determines the charge status and presence of the medical device, the anesthesia control system proceeds to step 124. In step 124, the anesthesia control system displays both the charge status and the presence status to the clinician in the dashboard 56 shown in Fig. 6. Based upon the displayed status, the clinician can determine whether the medical device, such as the video laryngoscope, is sufficiently charged and is in a location near the anesthesia device and whether the anesthesia procedure can begin. In an alternate step 126, the anesthesia control system can determine whether a replacement battery is available and the charge status on the replacement battery. Although a replacement battery is not required to begin an anesthesia procedure, step 126 provides an additional step for the clinician to carry out prior to beginning an anesthesia procedure.

In addition to receiving the presence status and charge status from the medical device, it is contemplated that other operational or function status information could also be received. This functional status information can be information received from the medical device that is related to the operational status of various systems in the medical device. This information could be generated as the result of an automatic or manually initiated self-test procedure.

This written description uses examples to disclose the invention, including the best mode, and also to enable any person skilled in the art to make and use the invention. Certain terms have been used for brevity, clarity and understanding. No unnecessary limitations are to be inferred therefrom beyond the requirement of the prior art because such terms are used for descriptive purposes only and are intended to be broadly construed. The patentable scope of the invention is defined by the claims, and may include other examples that occur to those skilled in the art. Such other examples are intended to be within the scope of the claims if they have features or structural elements that do not differ from the literal language of the claims, or if they include equivalent features or structural elements with insubstantial differences from the literal languages of the claims.

Unless otherwise specified or limited, the terms "mounted," "connected," "linked," "supported," and "coupled" and variations thereof are used broadly and encompass both direct and indirect mountings, connections, supports, and couplings. Further, unless otherwise specified or limited, "connected" and "coupled" are not restricted to physical or mechanical connections or couplings. As used herein, unless otherwise limited or defined, discussion of particular directions is provided by example only, with regard to particular embodiments or relevant illustrations. For example, discussion of "top," "bottom," "front," "back," "left" or "right" features is generally intended as a description only of the orientation of such features relative to a reference frame of a particular example or illustration. Correspondingly, for example, a "top" feature may sometimes be disposed below a "bottom" feature (and so on), depending on the position of the element and the frame of reference. Additionally, use of the words "first," "second", "third," etc. is not intended to connote priority or importance, but merely to distinguish one of several similar elements or machines from another.

## Claims

1. An anesthesia machine operable to deliver anesthesia to a patient and usable in concert with a medical device, the anesthesia machine comprising:
a main body;
a display screen mounted to the main body for presenting information to a user; and
a medical device holder associated with the main body for receiving and charging the medical device,
wherein status information related to the medical device is displayed on the display screen.

2. The anesthesia machine of claim 1 wherein the status information includes at least state of battery charge and presence of the medical device at the anesthesia machine.

3. The anesthesia machine of claim 2 wherein the medical device is a video laryngoscope.

4. The anesthesia machine of claim 1 wherein the main body includes at least one drawer that is movable into and out of the main body and the medical device holder is included within the at least one drawer.

5. The anesthesia machine of claim 4 wherein the medical device holder is an insert received within the at least one drawer, wherein the insert receives a supply of electrical power from the anesthesia machine.

6. The anesthesia machine of claim 5 wherein the insert includes a device charging holder for the medical device and at least one battery charging receptacle for receiving a replacement battery for the medical device.

7. The anesthesia machine of claim 6 wherein the anesthesia machine further comprises a connectivity module for receiving the status information from the insert, wherein the status information includes state of charge of the medical device, presence of the medical device and state of charge of the replacement battery.

8. A medical device holder for use with an anesthesia machine having a main body and a display screen, the medical device holder comprising:
a power connection to receive electrical power from the anesthesia machine; and
a device charging holder configured to receive a medical device and charge the medical device while the medical device is received by the device charging holder.

9. The medical device holder of claim 8 further comprising at least one battery charging receptacle configured to receive and charge a replacement battery and for the medical device.

10. The medical device holder of claim 8 wherein the medical device holder is in communication with the anesthesia machine to communicate status information for display on the display screen.

11. A method of operating an anesthesia machine in concert with a medical device, the method comprising the steps of:
determining a charge status of the medical device;
determining a presence status of the medical device relative to the anesthesia machine; and
displaying the charge status of the medical device and the presence status of the medical device relative to the anesthesia machine.

12. The method of claim **11** further comprising the steps of:
providing a medical device holder on or in a main body of the anesthesia machine, the medical device holder being configured to receive and charge the medical device; and
communicating the charge status and the presence status from the medical device holder to the anesthesia machine.

13. The method of claim 12 wherein the medical device holder includes a device charging holder and at least one battery charging receptacle configured to receive and charge a replacement battery.

14. The method of claim 11 wherein the charge status and the presence status are determined automatically.

15. The method of claim 11 further comprising the step of prompting the user to enter the presence status and the charge status as part of a checkout procedure for the anesthesia machine.
